Europäisches Patentamt

**European Patent Office**

**Office européen des brevets**

(19)

(11) Publication number: **0 173 375**
A1

(12) # EUROPEAN PATENT APPLICATION

(21) Application number: **85201228.5**

(22) Date of filing: **22.07.85**

(51) Int. Cl.⁴: **G 01 N 33/52,** G 01 N 33/563

(30) Priority: **23.07.84 US 633466**

(43) Date of publication of application: **05.03.86**
**Bulletin 86/10**

(84) Designated Contracting States: **AT BE CH DE FR GB IT
LI NL SE**

(71) Applicant: **POLAROID CORPORATION, 549 Technology
Square, Cambridge, Massachusetts 02139 (US)**

(72) Inventor: **Inbar, Shai, 198 Rawson Road, Brookline
Massachusetts 02146 (US)**
Inventor: **Huyffer, Paul S., 12 Andersen Drive, West
Boxford Massachusetts 01885 (US)**

(74) Representative: **Urbanus, Henricus Maria, Ir. et al, c/o
Vereenigde Octrooibureaux Nieuwe Parklaan 107,
NL-2587 BP 's-Gravenhage (NL)**

(54) **Novel assay product and process.**

(57) An assay device for determining the presence of analyte
(e.g. antigens and/or haptens) in a liquid sample comprising a
first (reagent) zone containing reference antigens and labeled
Fab fragments which are capable of reacting immunologically
with said analyte and said reference antigens, said Fab frag-
ments being positioned in said first zone such that they will be
removed therefrom when reacted with analyte passing through
said first zone but will not be removed from said first zone in the
absence of analyte; and a second (reaction) zone wherein the
reaction product of said labeled Fab fragment-analyte passes
after removal from said first zone and in which said label or a
reaction product thereof emits a detectable signal. The detect-
able signal emitted in said second zone may, for example, be a
color produced by reaction with a chromogenic material in said
second zone or it may, for example, be electromagnetic radiation,
e.g. chemiluminescence or fluorescence produced by interaction
with a reagent in said second zone.

NOVEL ASSAY PRODUCT & PROCESS

BACKGROUND OF THE INVENTION

U.S. Patent No. 4,446,232 issued May 1, 1984 to Lance A. Liotta relates to enzyme immunoassays which are said to be an improvement over enzyme-linked immuno-absorbant assays (ELISA) in that they require no dilution, no washing steps, and one short incubation period. The assay is in the form of a dry, layered test strip which forms a color reaction when exposed directly to the test fluid. This color reaction can be read visually, or with an instrument such as a spectrophotometer.

As described in the patent, the assay device is constructed in three distinct layers: (1) a first (outer) layer containing enzyme-linked antibodies; (2) a second (intermediate) layer containing immobilized antigens; and (3) a third layer containing a bound color-forming reagent, i.e. a material which reacts with an enzyme to produce a color.

Briefly, the assay device functions as follows: antigen in the sample combines with the enzyme-linked antibody in the first layer and the bound antibody-antigen complex thus formed diffuses through the second layer to the third where the enzyme label reacts with the color-forming reagent to produce a color as the detectable signal; whereas unbound enzyme-linked antibody in the first layer will diffuse to the second layer where it will combine with the immobilized antigen in that layer and will thus be prevented from diffusing to the third layer.

In essence, therefore, the assay is predicated upon the ability of antigen in the sample to permit the enzyme-linked antibody to reach the third layer to form a color. Optimally, for maximum quantitative efficiency, any antibody reacting with the antigen in the sample should reach this third layer and not be "tied up" in the second bound antigen-containing layer.

While the aforementioned assay affords distinct advantages as an assay system, as mentioned in the patent, the efficiency of the patented system in providing quantitative test results is appreciably lessened by antibodies which have reacted with antigen in the first layer still possessing free binding sites permitting them to bind to immobilized antigen in the second layer and thus becoming trapped so they never reach the third layer to contribute to the color signal produced in the third layer.

The present invention in a sense can be said to be an improvement over the teachings of U.S.P. 4,446,232, the essence of the invention being to obviate the aforementioned problem and thereby increase the efficiency of the assay to provide a detectable signal, the quantum of which is a function of the amount of analyte present in the sample. However, the present invention is somewhat broader in that it is not restrictd to the use of enzyme labels to produce a color.

BRIEF DESCRIPTION OF THE INVENTION

According to the present invention, the aforementioned assay system is markedly improved by employing enzyme- or other label-linked Fab fragments in lieu of antibodies in the first layer.

BRIEF DESCRIPTION OF THE DRAWINGS

Fig. 1 is a fragmentary, enlarged diagrammatical illustration showing stepwise the formation of a color signal in accordance with the invention described of U.S.P. 4,446,232;

Fig. 2 is a graph showing the kinetic equations of the system of Fig. 1, as solved numerically on the computer; and

Fig. 3 is a diagrammatic view of the improved assay device in accordance with this invention.

DETAILED DESCRIPTION OF THE INVENTION

As mentioned previously, U.S.P. 4,446,232 relates to an improvement over the ELISA enzyme-labeled assays in that the use thereof requires no dilution, no washing steps and one short incubation period. The assay is in the form of a dry, layered test strip which forms a color reaction when exposed directly to the test fluid. The strip automatically performs the dilution steps required for quantitation and separates the bound antibody from the free antibody. The color reaction can be read visually, or with an instrument, such as a spectrophotometer. In addition, it can be fabricated in strip form and employed as a dipstick for rapidly detecting an antigen, such as a drug or hormone in urine. An example of a specific application would be the rapid detection of a drug overdose, or as a home pregnancy test.

As stated in the patent, in one aspect the invention concerns a device for determining the presence of antigens which comprises a first zone containing antigens and enzyme linked antibodies which are capable of immunologically reacting with these antigens, the antibodies being positioned in the first zone such that they will be removed from the first zone when reacted with antigens passing through the first zone but will not be removed from the first zone in the absence of such antigens, and a second zone containing material capable of reacting with the enzyme-linked antibodies to produce a color forming reaction which indicates the presence of these antibodies.

It is further stated that in another aspect, the patented invention is directed to a unique method for determining the presence of antigens in a biological fluid which comprises bringing the fluid into contact with a device having a first zone containing antigens and enzyme-linked antibodies which are capable of immunologically reacting with the antigens, the antibodies being positioned in the first zone such that they will be removed from the first zone when reacted with antigens passing through the first zone but not removed from the first zone in the absence of such antigens, and a second zone containing material capable of reacting with the enzyme-linked antibodies to produce a color forming reaction which indicates the presence of the antibodies; allowing the fluid to permeate the device or matrix; and observing the presence or absence of any color change in the second zone, thereby to determine the presence or absence of the antigen in the fluid which is the subject of the test.

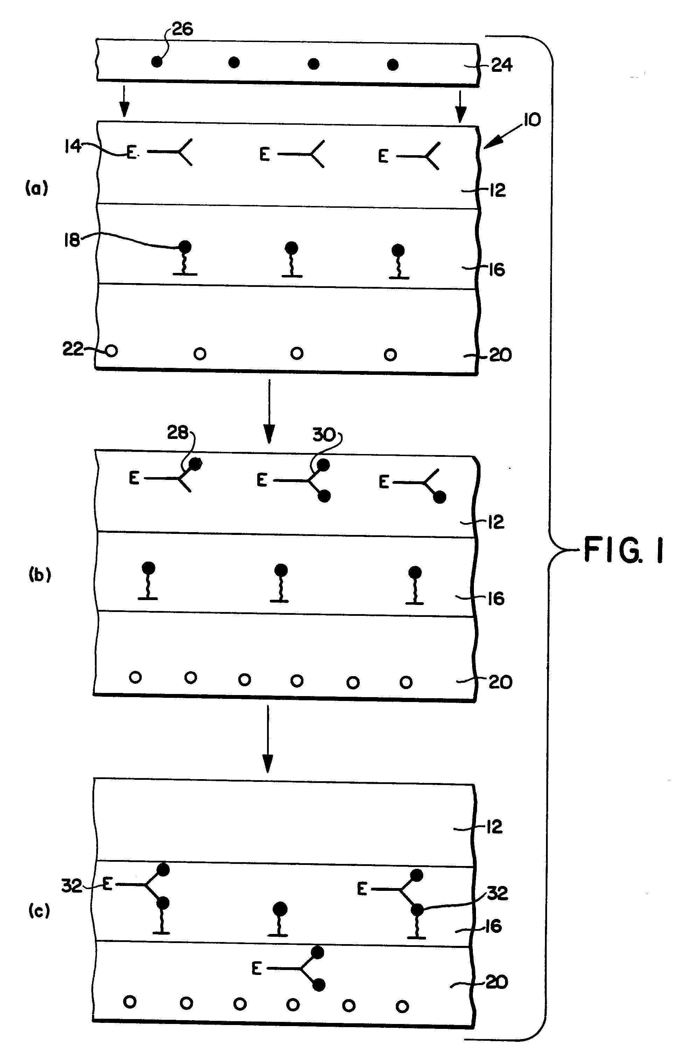

Fig. 1 of U.S.P. 4,446,232 illustrates the invention. As seen from this figure and the description thereof, the device is constructed in three distinct layers: a first layer containing soluble enzyme-linked antibodies; a second layer containing bound antigens; and a third layer containing a material which reacts with the enzyme to produce a color.

In operation, the fluid sample containing the antigen to be tested, is placed in contact with the first layer. The free antigen in the test sample diffuses into the second layer and then into the third layer. The free antigen in the sample competes with the immobilized bound reference antigen in the second layer for combining with enzyme-linked antibody. If the enzyme-linked antibody combines with free antigen, it will freely diffuse into the third layer and produce a color reaction. If the fluid sample contains no antigen, all the enzyme-linked antibody will have free binding sites and will combine

0173375

with the immobilized antigen in the second layer. Enzyme-linked antibody which combines with the immobilized antigen in the second layer will not diffuse into the third layer, and no color reaction will be produced. The difference in the amount of substrate degraded (and intensity of color produced) is proportional to the amount of antigen in the test sample. For a given amount of enzyme-labeled antibody in the first layer, the sensitivity of the device is determined by the amount of reference antigen present in the second layer. Typically, a test device would contain a series of sandwich composites, each with a different amount of reference antigen. The series of reference antigen concentrations would have been previously determined to span a range of sensitivity, appropriate for the test solution being measured.

To recapitulate, in accordance with the disclosure of U.S.P. 4,446,232, enzyme-labeled antibody is dispersed in the upper layer, antigen to the same antibody is immobilized in the second layer and a color forming compound, activated by the enzyme label on the antibody, is found in the third layer. When a test sample containing the antigen is applied to the upper layer, it reacts with a proportional part of the enzyme-linked antibodies in that layer and the reaction mixture is transferred by diffusion to the second layer.

In accordance with the intentions of the patentee, the reaction mixture should continue through the second layer to the third layer to form the desired color signal. However, this reaction mixture will contain a substantial majority of antibodies which still possess free binding sites and can therefore bind to the immobilized reference antigen in the second layer and become trapped. In other words, as is stated in the patent, enzyme-linked antibody which combines with immobilized antigen in the second layer will not diffuse into the third layer.

Thus, it is important to understand that only antibodies which have fully reacted with the sample antigen in the first layer should pass through the second layer and into the third layer where they can activate the chromogenic material and color is provided. Although it is said that the color signal thus generated is directly proportional to the amount of the antigen that was present in the test sample, it may perhaps be more accurate to say that the color signal that is generated is in fact directly proportional to the amount of enzyme-linked antibody that diffuses to the third layer.

The present invention can be said to have been initiated by the appreciation of the necessity in the aforementioned patented system for forming 2:1 complexes of antigen-antibody. Antibodies which have reacted with only one antigen molecule still possess a free binding site and may become trapped in the second layer. Consequently, much of the potential signal will be lost.

Fig. 1 illustrates schematically in a sequence of three steps, (a), (b) and (c). The aforementioned problem is generating the optimum color signal.

As shown therein, element 10 comprises a top layer 12 of enzyme-linked antibody 14; an intermediate layer 16 of immobilized antigen 18; and a bottom layer 20 of chromogenic material 22. [For simplicity of illustration of the problem, in the fragmentary sketch only a few few of the present reagents 14, 18, and 22 are shown.]

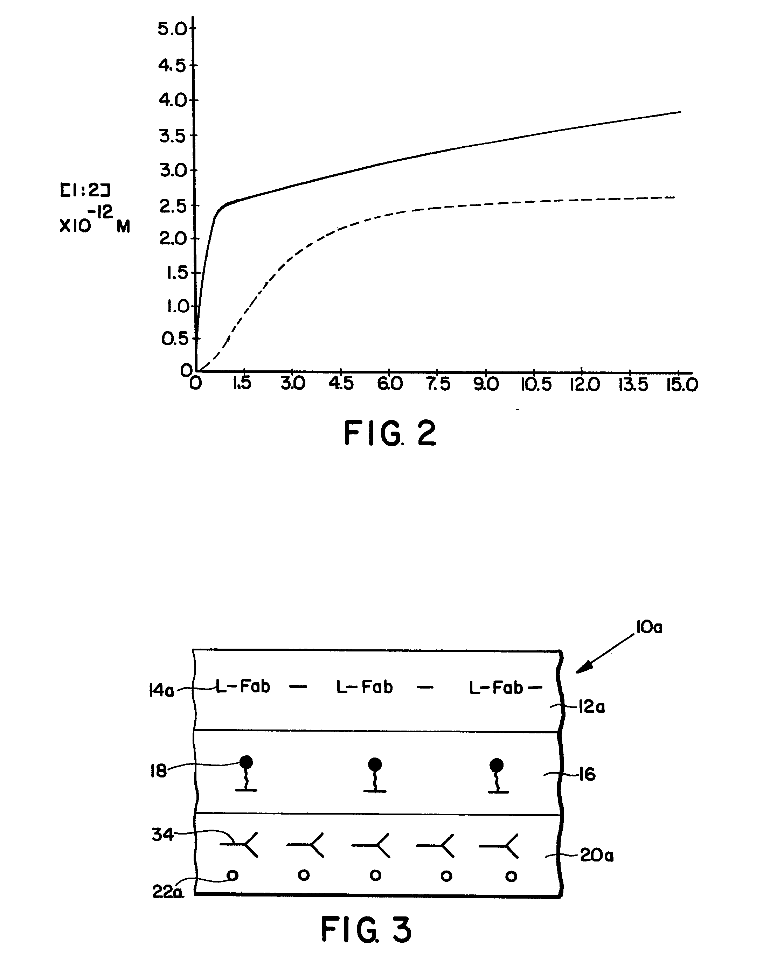

In Fig. 3(a), a sample 24 containing antigens 26 is applied to the top layer 12, e.g. with the aid of a suitable spreader element or the like.

In Fig. 3(b) which can be regarded as the initial stage of the color-generating reaction, antigen in the sample reacts immunologically with the antibody in top layer 12.  As illustrated, two of the resulting complexes 28 have antigen bound to only one of the binding sites of the antibody; and only one of the three illustrated antibodies provides a complex 30 containing antigens at both binding sites.

As seen in Fig. 3(c) illustrating the next or completion stage of the sequence, complexes 28 having a free or available binding site become trapped in layer 16 where the free binding site reacts immunologically with reference antigen in layer 16.  Thus, of the three illustrated enzyme-linked antibodies which should in theory diffuse to layer 20 to react with the chromophoric material 22, only the one complex 30 from the reaction in layer 12 in fact reaches its desired destination in the color signal-generating layer 20.

While for purposes of illustration, only two out of the three shown antibodies become trapped in the intermediate layer and thus fail to reach their destination to contribute to the intensity of the color signal, actually the problem is considered to be on the order of a magnitude worse.

Calculations show that under realistic conditions, this loss, as illustrated above, amounts to most of the theoretical signal.

Association rate constants for typical antibody-antigen reactions are in the order of $10^5$ to $10_6 . 1$ mole $^{-1} sec^{-1}$.  For realistic low limit of detection of antigens ($< 10^{-9}$M), one must use a large excess of antibody to assume reasonable reaction time.  Calculations below show that under such conditions, the fraction of 1:2 complex in the equilibrium mixture is very small.

Formation of 1:2 complex can be assumed if excess of sample antigen is used compared to the enzyme-linked antibody in the first layer.  This, however, also means low sensitivity in the assay, as large concentrations of antigen are required to obtain positive results.

The reactions by which the 1:1 and 2:1 antigen-antibody complexes are formed may be described by the following sequence:

$$Ab + Ag \xrightarrow[K_{-1}]{K_1} AbAg \qquad K_1$$

$$AbAg + Ag \xrightarrow[K_{-2}]{K_2} AbAg_2 \qquad K_2$$

The reactions occur in solution and for given binding constants the equilibrium state can be evaluated on the computer. Typical binding constants for antibody-antigen reactions are of the order $K=10^9$. Accordingly, we can take this constant as the basis for calculations. We can also assume that the binding constants are the same for both reactions. [It should be noted, however, that the results will be changed very little (less than one percent) if higher binding constants are used.]

The concentration of antibodies in the top layer must be well in excess of the lower limit of test antigen in order to ensure both reasonable reaction time and a good dynamic range for the test. Taking the lower limit of antigen as $10^{-9}M$ and calculating for antibody in 100 and 1000 fold excess, the results are set forth below in Table 1:

| [Ag] sample | [Ab] upper layer | 1:1 complex | 1:2 complex |
|---|---|---|---|
| $1 \times 10^{-9}$ | $1 \times 10^{-6}$ | $0.99 \times 10^{-9}$ | $0.001 \times 10^{-9}$ |
| $3 \times 10^{-9}$ | " | $2.98 \times 10^{-9}$ | $0.009 \times 10^{-9}$ |
| $5 \times 10^{-9}$ | " | $4.95 \times 10^{-9}$ | $0.025 \times 10^{-9}$ |
| $10 \times 10^{-9}$ | " | $9.82 \times 10^{-9}$ | $0.097 \times 10^{-9}$ |
| $30 \times 10^{-9}$ | " | $28.00 \times 10^{-9}$ | $0.830 \times 10^{-9}$ |
| $50 \times 10^{-9}$ | " | $46.00 \times 10^{-9}$ | $2.220 \times 10^{-9}$ |
| $1 \times 10^{-9}$ | $1 \times 10^{-7}$ | $0.97 \times 10^{-9}$ | $0.009 \times 10^{-9}$ |
| $3 \times 10^{-9}$ | " | $2.81 \times 10^{-9}$ | $0.081 \times 10^{-9}$ |
| $5 \times 10^{-9}$ | " | $4.50 \times 10^{-9}$ | $0.215 \times 10^{-9}$ |
| $10 \times 10^{-9}$ | " | $8.36 \times 10^{-9}$ | $0.773 \times 10^{-9}$ |
| $30 \times 10^{-9}$ | " | $19.50 \times 10^{-9}$ | $5.100 \times 10^{-9}$ |
| $50 \times 10^{-9}$ | " | $26.60 \times 10^{-9}$ | $11.400 \times 10^{-9}$ |

Equilibrium Conditions for the Upper Layer
Whole Antibodies, $K_1 = 1 \times 10^9$; $K_2 = 1 \times 10^9$

The results show clearly that at the lower limit of antigen used, almost all of the antigen is trapped in a 1:1 complex which, as explained above, does not generate a signal. Only a small fraction will form a 2:1 complex that can pass through the second layer without being trapped and thus generate color in the third layer. The higher the excess of antibody used, the lower is this fraction ($\sim$0.1% vs. $\sim$1.0%). Yet, on the other hand, reducing the antibody excess will in turn result in a reduction of signal due to incomplete reaction in the antibody-containing layer, as well as loss or reduction of the test dynamic range.

Another inherent problem to be considered in the aforementioned system is the possibility of agglutination in the upper layer due to the large excess of divalent antibodies. Any such agglutination will result in a still further loss of signal.

The foregoing discussion assumes equilibrium conditions are obtained in the upper (antibody) layer. However, this is not necessarily true. Typical association rates for antigen-antibody reactions are on the order of $10^5-10^6$ l./mole sec. This means that for an antibody concentration of $1 \times 10^{-7}M$ in the upper layer, the reaction time is on the order of 10-100 seconds for the first association. The time required for the generation of the 2:1 complex is much longer than that, since the antigen concentration is immediately depleted by the first reaction. Since the illustrative test example in the patent indicates (Col. 7) that the immunological reaction product containing the enzyme label starts arriving in the third layer in about ten to thirty seconds, the residence time in the first layer is insufficient to attain equilibrium. The calculations presented in Table 1 give the upper limit for the signal, but this is clearly not achieved in practice.

The kinetic equations for the system were solved numerically on the computer and typical results are presented in Fig. 2 of the drawings.

In this graph, the X axis shows the time in minutes and the Y axis shows the concentration of 2:1 enzyme-labeled antibody-antigen complex in the top layer for an assay in which the initial or starting concentration of antigen is $1 \times 10^{-9}M$ and $1 \times 10^{-7}M$ antibody, the equilibrium concentration being $9.9 \times 10^{-12}M$. [See Table 1.]

With respect to the kinetics of the antibody-antigen binding shown in the graph:

for the solid line curve

$$k_1 = 1 \times 10^6 \qquad k_2 = 0.5 \times 10^6;$$

and for the dotted line curve

$$k_1 = 1 \times 10^5 \qquad k_2 = 0.5 \times 10^5$$

In either instance, it will be seen that most of the obtainable 2:1 complex occurs within the first minute or so, and then the curve tends to flatten out. Eventually, weeks perhaps, equilibrium may be attained. However, this is not possible within the time span for any feasible assay.

It will therefore be seen that the kinetics show two distinct phases. At the first stage, the 2:1 complex is formed relatively fast, since there is still a considerable amount of free antigen present to react with the initially formed 1:1 complex. Later, the concentration of free antigen is drastically depleted (most of it being bound as the 1:1 complex) and the reaction to form 2:1 complex becomes very slow. Accordingly, within the time span contemplated by the patentee, only $\sim$10-20% of the equilibrium limit is achieved assuming fast rate constants, while no more than $\sim$1-2% is obtained for the slower rate constants.

In accordance with the present invention, the aforementioned problems may be materially lessened, making it possible to improve sensitivity by some three orders of magnitude, simply by replacing the whole antibodies in the top layer with Fab fragments.

As is well known, the IgG antibodies can be split, e.g. by papain, into three fragments. Two of these are identical and can combine immunologically with antigen as would the whole antibody. They are univalent antibody fragments and are given the nomenclature Fab ("fragment antigen binding"). The third fragment has no power to combine with antigen and is termed Fc ("fragment crystallizable", obtainable in crystalline form).

In general, any Fab fragments specific to the analyte to be assayed may be employed in the practice of this invention. However, the label attached to the Fab fragment need not be an enzyme which can react with a chromogenic substance to form a color. Thus, in accordance with this invention, the label may be any of the per se known ones which can provide a detectable signal or is the precursor of a substance which can provide a detectable signal.

Accordingly, the novel assay elements of this invention comprise the essential layers illustrated in Fig. 3. As shown therein, the assay element 10a comprises a first layer 12a containing labeled Fab fragments 14a to which the sample is applied; an intermediate layer 16 of bound antigen 18 immunologically reactable with the labeled Fab fragments 14a in the top layer and a third layer 20a containing a reagent 22a which can react with the labeled Fab-analyte complex diffusing thereto to provide the detectable signal, the above essential layers being in fluid contact with one another.

It will be appreciated that the assay elements of this invention may include other layers or elements performing specific desired functions and the structure shown in Fig. 3 for purposes of illustration includes only the elements which are essential to the practice of the assay system.

For example, the aforementioned essential layers may be and preferably are contained on a suitable transparent support or sheet (not shown) through which the detectable signal can be discerned. Since the assay element is intended for liquid samples such as whole blood which have a color detracting from the signal, a suitable opaque layer hiding the applied sample is preferably disposed between layer 20a and layer 12a. This opaque layer preferably comprises a white material, e.g. a pigment such as titanium dioxide in order to provide a white reflective background for viewing or recording the detectable signal.

Other layers or elements performing specific desired functions may also be included. By way of further illustration, the novel assay system of this invention may be utilized in the assay products which are described and claimed in the copending application of Ernest W. Long, Serial No. 629,445, filed July 10, 1984. The assay products of this copending application comprise, as essential components an assay element predicated upon any desired system for providing a detectable signal and encoded means containing indicia pertaining to the operating conditions for obtaining and recording the detectable signal, which product can then be employed in conjunction with an analyzer or detector for a plurality of different assays and which contains means for reading and translating the encoded means to obtain the proper processing and recording conditions for the particular assay element. As further disclosed in the aforementioned copending application, the assay product may further include a rupturable container of fluid of the type per se well known in the art of one-step diffusion transfer photography, which fluid includes at least one reagent desired for the particular assay system, the container being so constructed and positioned so that upon rupturing, its fluid contents are discharged at the desired interface on or within the assay component.

Since the label "L" on the Fab fragments of this invention is not restricted to enzymes which provide a color, as are disclosed in U.S.P. 4,446,232, it is visualized that in some embodiments which one skilled in the art may contemplate, it may be desirable or expedient to include a reagent which reacts with, initiates or catalyzes formation of the detectable signal with the particular label employed. In this event, the rupturable container should be positioned so as to be capable of applying its contents to layer 20a.

As mentioned earlier, employment of the univalent Fab fragments in lieu of whole antibodies, in accordance with this invention, may improve sensitivity and thereby increase the signal by some three to four orders of magnitude. While this improvement may of course be of lesser consequence in simple qualitative assays, it is of very great improvement in dynamic range in assays where quantitative analysis for detection of analyte is contemplated.

The reason why substitution of Fab fragments will provide the above-noted improved signal should be readily apparent in view of the foregoing discussion of the problems with using whole antibodies.

Initially, it will be noted that the signal-generating complex formed in the top layer is a 1:1 complex, making it possible to realize the full potential of the signal-generating complex. None of the 1:1 complex so formed will become undesirably trapped in the intermediate layer for the reasons heretofore discussed. Since the 1:1 complex formed with Fab fragments has no free binding site, it will be completely transferred to the third layer, contributing to the intensity of the generated signal.

It should also be noted that the use of a large excess of Fab fragment in the upper layer will not lead to loss of signal or to agglutination.

Another advantage is also apparent. As seen from Table 1, when whole antibodies are used, the dose response curve is highly nonlinear. However, as seen from Table 2, below, as calculated with the computer, the use of Fab fragments leads to a nearly linear dose response curve which can be advantageous if the whole dynamic range of the test is used.

| [Ag] sample | (Fab) upper layer | 1:1 complex |
|---|---|---|
| $1 \times 10^{-9}$ | $1 \times 10^{-6}$ | $1.00 \times 10^{-9}$ |
| $3 \times 10^{-9}$ | " | $2.99 \times 10^{-9}$ |
| $5 \times 10^{-9}$ | " | $4.99 \times 10^{-9}$ |
| $10 \times 10^{-9}$ | " | $9.99 \times 10^{-9}$ |
| $50 \times 10^{-9}$ | " | $49.97 \times 10^{-9}$ |
| $1 \times 10^{-9}$ | $1 \times 10^{-7}$ | $1.00 \times 10^{-9}$ |
| $3 \times 10^{-9}$ | " | $2.97 \times 10^{-9}$ |
| $5 \times 10^{-9}$ | " | $4.94 \times 10^{-9}$ |
| $10 \times 10^{-9}$ | " | $9.89 \times 10^{-9}$ |
| $50 \times 10^{-9}$ | " | $49.00 \times 10^{-9}$ |

TABLE 2

Equilibrium Conditions for the Upper Layer
Fab Fragments, $K_1 = 1 \times 10^9$

Beneficial effects in kinetic control should also be attained with the use of Fab fragments. Not only will the signal generating complex be formed by the relatively faster initial process, but use of higher Fab fragment concentrations is permissible, as explained previously, making possible faster reaction rates.

The particular Fab fragments which can be employed in the practice of this invention may, of course vary widely, in accordance with the availability of antibodies, naturally occurring or mono- or polyclonol, immunologically specific to the particular analyte in the sample. Procedures for obtaining Fab fragments from antibodies is of course well known in the art and reported in the literature.

By way of illustration, Fab fragments of the antibodies disclosed in the aforementioned U.S.P. 4,446,232 may be utilized, i.e. rabbit antisera to human hCG (human chorionic gonadotrophin); and peroxidase conjugated anti-HBA (human hepatitis B antigen. As examples of other useful Fab fragments, mention may be made of those disclosed, for example, in U.S.P. 4,235,869 issued November 25, 1980 to Moshe Schwarzberg, i.e. Fab anti-hIgG (human immunoglobulin G); and Fab anti-hIgM (human immunoglobulin M). Other Fab fragments which may be employed and are hence contemplated by this invention will be readily apparent, as will their preparation.

As mentioned, Fab fragments are well known, as is their preparation. A description may be found, for example, in "Advanced Immunochemistry", Eugene Day, the Williams and Wilkins Company, 1972, pp 88 et seq. In general, they can be prepared by digestion with a peptidase, such as papain, trypsin or pepsin. Other treatments may be involved such as reduction, substitution, e.g. aminopthylation, carboxyalkylation and the like. The resulting monovalent fragment is characterized as retaining a high degree of the specificity and binding constant of the intact antibody.

By way of further illustration, reference may be made to the preparation of Fab fragments in accordance with the disclosure of the aforementioned U.S.P. 4,235,869.

## EXAMPLE 1
### Preparation of Fab anti-hIgG
[according to Example 1 of U.S.P. 4,235,869] 50 ml of sheep serum containing anti-hIgG is precipitated with 50 ml of saturated ammonium sulfate.

0173375

The resulting precipitate is then centrifuged for about twenty minutes at about 48,2000 x g and then resuspended in PBS (0.04 M phosphate in saline, pH 7.2). The suspension is dialyzed against the same buffer solution for two days to the final concentration. A 4.41 ml aliquot of this protein solution in PBS (400 mg of protein) is combined with 2 ml of PBS containing about 0.05 M cysteine and 0.02 M EDTA (ethylenediamine tetraacetic acid), 13.59 ml PBS and 4 mg papain (Sigma, 0.16 ml of a 25 mg/ml suspension). [The substrate solution is warmed to about 37° C. before the enzyme addition and the mixture is allowed to incubate overnight with stirring while maintaining the temperature at 37°C. After adjusting the pH to 6.5 with 1 N HCl, 2.2 ml of a 0.055 M iodoacetamide solution is then added. The resulting mixture is then incubated for five minutes at room temperature and dialysis initiated against 0.01 M phosphate, pH 6.5. Any slight precipitation which is formed may be removed by centrifugation and the resulting clear solution then dialyzed against 0.15 M sodium chloride. After dialysis, the material is made 0.05 M in zinc sulfate by the addition of 0.25 M zinc sulfate and allowed to sit at room temperature for two hours. The resulting precipitate is then centrifuged for twenty minutes at 48,200 x g, after which sodium EDTA is added slowly with stirring to provide a one percent solution. The resulting precipitate is removed by centrigugation and the solution then dialyzed against PBS to provide an expected yield of about 147 mg. of the Fab fragment.

## EXAMPLE 2
### Preparation of Fab anti-hIgM
[according to Example 5 of U.S.P. 4,235,869]

Solid disodium monoacid phosphate is added to 30 ml of sheep anti-serum containing anti-hIgM to bring the pH to 8. The solution is then made 18% in sodium sulfate and stored for twenty minutes at room temperature. The precipitate which is formed is separated by centrifugation and redissolved in 15 ml of 0.1 M potassium phosphate buffer, pH 8. The resulting mixture is centrifuged at room temperature, the solution made 12% in sodium sulfate and the precipitate again collected by centrifugation. The protein precipitate is redissolved in 0.1 M sodium acetate buffer, pH 5.4, and dialyzed overnight against the same buffer. The resulting solution is clarified by centrifugation (about thirty minutes at 10,000 rpm.) The resulting mixture is then incubated with 2.5 mg of papain at 37°C for about ten hours. Sodium iodoacetate in water (0.1 ml) is added to this mixture to adjust the final concentration to 2.3 mM. After one hour at room temperature, the solution is washed to remove small peptides on Amicon membrane PM10 with 0.05 M potassium phosphate buffer, pH 8 (15 ml) and then concentrated down to 4 ml. The resulting solution may then be clarified by centrifugation to provide an expected yield of about 18.5 mg/ml of the Fab solution.

As mentioned previously, the label for the Fab fragment in accordance with this invention may be any of the color-providing enzyme labels disclosed in U.S.P. 4,446,232, but is not so restricted. For example, the enzyme label may be a reagent such as horseradish peroxidase, alkaline phosphatase or beta-galactosidase which is reactable with a color-forming agent such as diaminobenzidine or p-nitrophenylphosphate in the presence of hydrogen peroxide to provide the desired color signal.

It may also be one of the per se known labels which emit a detectable signal. Of particular interest are labels which emit detectable electromagnetic radiation.

Thus, by way of further illustration it may be of the class generally known as fluorescers or fluorophors. Such compounds for the most part absorb light above 300 nm, ideally above 400 nm and preferably have extinction coefficients of at least $10^3$, most preferably at least $10^4$ above the indicated wavelengths.

As examples of compounds which may be employed as fluorescers, mention may be made of xanthene dyes, including the fluoresceins derived from 3,6-dihydroxy-9-phenylxanthhydrol; and rosamines and rhodamines, derived from 3,6-diamino-9-phenylxanthhydrol. The rhodamines and fluoresceins have a 9-o-carboxyphenyl group and are derivatives of 9-o-carboxyphenylxanthhydrol.

Other dyes which may be used as fluorescers include:
3-phenyl-7-isocyanatocoumarin, acridines, such as 9-isothiocyanatoacridine and acridine orange N-(p-(2-benzoxazolyl)phenyl)maleimide; benzoxadiazoles, such as 4-chloro-7-nitrobenzo-2-oxa-1,3-diazole and 7-(p-methoxybenzylamino)-4-nitrobenzo-2-oxal,2-diazole; stilbenes, such as 4-dimethylamino-4'-isothiocyanatostilbene and 4-dimethylamino-4'-maleimidostilbene N,N'-dioctadecyl oxacarbocyanine p-toluenesulfonate; pyrenes, such a 8-hydroxy-1,3,6-pyrenetrisulfonic acid, and 1-pyrenebutyric acid; merocyanines e.g. merocyanine 540; rose bengal; 2,4-diphenyl-3(2H)-furanone; cyanines; anthraquinones; porphyrins; triarylmethanes; etc.

Another class of useful labels are luminescent compounds which in the presence of another compound or activator emit light.

Luminescence may be simply defined as the emission of visible or invisible radiation which is the result of a chemical reaction. In chemiluminescence the source of the energy that is producing molecules in an excited state is the energy of a chemical reaction and the decay from the excited state back to the ground state is accompanied by emission of light (luminescence). Analytically, the types of luminescence that have engendered the most interest are chemiluminescence and bioluminescence. The latter is the name given to a special form of chemiluminescence found in biological systems, in which a catalytic protein increases the efficiency of the luminescent reaction.

The mechanism of organic chemiluminescence in solution can be summarized as involving three stages: (1) preliminary reactions to provide the key intermediate; (2) an excitation step in which the chemical energy of the key intermediate is converted into excitation energy; and (3) emission of light from the excited product formed in the chemical reaction. In reactions in which a fluorescent compound is added to enhance the chemiluminescent emission, an efficient energy transfer occurs and the resulting chemiluminescence is known as "sensitized chemiluminescence."

Chemiluminescent reactions systems require an oxidant (usually hydrogen peroxide) which can be produced, as in step (1) above by preliminary reactions which produce the key intermediate. They also require a catalyst, for example, microperoxidase, heme, hemoglobin or cobalt. Additionally, they require a chemilumingenic compound which receives the excitation energy from the key intermediate (e.g. hydrogen peroxide) by the effect of the catalyst. When the chemilumingenic compound returns to its base state from the high energy excited state a release of energy is in the form of detectable light.

As examples of chemiluminescent compounds useful as labels in assays, mention may be made of diacylhydrazides such as 5-amino-2,3-dihydro-1,4-phthalazinedione(luminol, 6-amino-2,3-dihydro-phthalazine-7,4-dione (isoluminol), annealated analogs of luminol, etc.; acridinium salts such as lucigenin (bis-N-methylacridinium nitrate, acridinium phenyl carboxylates, etc.; diaryl oxalates such as bis(trichlorophenyl)oxalate.

Various luminescent immunoassays, luminescent enzyme immunoassays, luminescent cofactor immunoassays, and luminescent enzyme-multiplied immunassays have all been reported in the literature and need not be discussed. In general, luminescent labels from the known state of the art which can be bonded to the Fab fragment in accordance with this invention will be apparent to those skilled in the art. For example, the list of chemiluminescent compounds disclosed in U.S.P. 4,235,869 as useful labels for Fab fragments may be employed.

By way of further illustrations, peroxidase enzyme, which will react with a color-forming reagent to produce a color, may be conjugated to the Fab fragment illustrated in Examples 1 or 2 by the standard periodate method, as mentioned in Example 1 of U.S.P. 4,446,232, followed by purification of the conjugate by conventional methods.

The following additional examples, taken from the aforementioned U.S.P. 4,235,869 illustrate the preparation of labeled Fab fragments in accordance with the invention.

## EXAMPLE 3

Preparation of fluoresceinisothiothiocyanate-
labeled Fab anti-hIgG
[according to Example 2 of U.S.P. 4,235,869]

4 ml of a Fab anti-hIgG slution [as prepared in Example 1] is adjusted to pH 8.75 with potassium monoacid phosphate. 1.4 mg. of fluoresceinisothiocyanate is then added and the mixture is allowed to stand for two hours at room temperature. The mixture is then separated and equilibrated with 0.05 M phosphate, pH 8 (0.05% sodium azide). The aryl fraction (which should have the characteristic yellow color) is then collected to provide the fluorescein-labeled Fab.

EXAMPLE 4

Preparation of Fluorescein-labeled Fab anti-hIgM
[According to Example 5 of U.S.P. 4,235,869]

Two ml. of the Fab solution prepared in Example 2 are brought to pH 9.0 by the addition of solid sodium carbonate. A solution of 1.5 mg of fluorescein isothiocyanate in 0.1 ml dry dimethylformamide is slowly added at room temperature over a period of thirty seconds, while stirring. After an additional two hours, the solution may be briefly clarified on a centrifuge and applied for separation into a column of Sephadex G-25 equilibrated with 0.05 M potassium phosphate buffer at pH 8.0. The yellow fluorescein-labeled Fab (first band eluted) is then collected.

In view of the foregoing illustrations and discussion of useful Fab fragments and labels, one skilled in the art may easily understand the preparation of useful embodiments of this invention. However, it is stressed that the invention is not directed to the use of specific reagents, but is instead directed to the arrangement of essential layers as described in connection with the accompanying drawing.

Thus, in one sense it can be said to be an improvement over the assay systems disclosed in U.S.P. 4,446,232 in the sense that Fab fragments are used in lieu of whole antibodies to provide a more efficient signal.

Since U.S.P. 4,446,232 only discloses enzyme labels which can produce a color, in another sense the present invention is broader than the invention disclosed in U.S.P. 4,446,232 in that, for purposes of the present invention, the kind of detectable signal that is emitted is immaterial. Accordingly, any of the per se known labels, e.g. as were heretofore employed in immunoassays, are herein contemplated.

The preparation of the Fab fragments from whole antibodies and, in turn, their conjugation with the chosen labels involve routine analytical procedures within the judgment and knowledge of the skilled worker and per se comprise no part of this invention. However, for purposes of illustration, procedures for the preparation of suitable labeled-Fab fragments were "borrowed" from the aforementioned U.S. Patents Nos. 4,235,869 and 4,446,232.

The preparation of the essential layers shown in Fig. 3 may follow the preparations of the analogous layers in U.S.P. 4,446,232, e.g. in terms of suitable thickness, matrices, etc.

In general, as in the patent disclosure, layers 12a, 16 and 20a will all comprise a porous matrix so the sample fluid may readily permeate therethrough. Suitable materials for fabricating these layers are well known in the art. They include, for example, interwoven fibers such as nitrocellulose or diazobenzyloxymethyl (DBM) paper. Nitrocellulose paper directly binds proteins and has been shown to be useful for immobilizing antigens. DBM matrix binds DNA, RNA, and proteins. Additionally, a porous gel can be utilized, e.g. polyacrylamide, agavose or collagen. The antigen in layer 16 can be trapped within the pores of the gel, or it can be cross-linked to the gel via amino groups of the antigen and carboxylic groups on the matrix. Further, particles or beads containing the bound antigen trapped within a cellulose or plastic fiber matrix can be utilized. For example, one may employ for layer 16 polyacrylamide beads, 5-10 microns in diameter, with antigen bound to the surface via a peptide bond. The beads are trapped within a cellulose filter matrix of pore size 1-2 microns.

Since the labels may vary according to the desire or whim of the practitioner or clinician, reagent 22a in layer 20a is not capable of precise definition. In any event, reagent 22a will comprise the reagent or combination of reagents needed to generate the signal from the labeled Fab-analyte complex which has diffused thereto and the selection of this reagent or combination of reagents will be well understood by those skilled in the art.

As mentioned earlier and discussed in U.S.P. 4,446,232, where reagent 22a is a color former to be used with enzyme labels to generate a color signal, the label may be horseradish peroxidase, alkaline phosphatase, beta-galactosidase, and the like. On the other hand, where the label is one which can react to emit electromagnetic radiation, reagent(s) 22a will be the oxidant, catalyst, etc. required for the reaction or energy transfer to emit the signal in accordance with per se known technology.

The thicknesses of the three essential layers are not critical and will of course vary in accordance with the proportions of selected ingredients contained therein. In general, of course, the laminate should be as thin as possible.

While the foregoing description constitutes the elements necessary to the practice of this invention, it is noted that one element shown in the illustrative Fig 3 has not yet been described, namely reagent 34 in layer 20a which is shown to be an antibody.

The format described previously, as well as that disclosed in U.S.P. 4,446,232, does not include mordanting in the third layer (layer 20a of the drawings). This absence can lead to further loss of signal. No driving force is present to direct diffusion

0173375

to this layer. Thus, the labeled complex will equilibrate among the layers and not be fully used. The use of a mordant for the diffusing labeled complex, e.g. an enzyme-labeled complex, will provide a clear benefit and a gain in signal by a factor of about 3 may be anticipated. While any mordant which can bind or fix the diffusing complex may be employed for this purpose, antibodies which can immunologically react, e.g. with free binding sites on the analyte are both known and preferred. Accordingly, the mordant 34 is shown for purposes of illustration to be an antibody.

The concept of employing mordants in the signal-producing layer of the aforementioned assay devices and those of U.S.P. 4,446,232 is the subject matter of our copending application, Serial No. (    ) filed          .

Since certain changes may be made without departing from the scope of the invention herein involved, it is intended that all matter contained herein or shown in the accompanying drawings shall be interpreted as illustrative and not in a limited sense.

What is claimed is:

0173375

## CLAIMS:

6984     1. An assay device for determining the presence of analyte in a liquid sample which comprises:

a first zone containing immobilized reference antigens and Fab fragments having linked thereto a label for providing a detectable signal on which is the precursor for forming a substance providing a detectable signal, said labeled Fab fragments being capable of reacting immunologically with said analyte and with said reference antigens, said labeled Fab fragments being positioned in said first zone such that they will be removed therefrom when reacted with analyte passing through said first zone but will not be removed from said first zone in the absence of analyte; and

a second zone wherein the reaction product of said labeled Fab fragments and said analyte passes after removal from said first zone and in which said label or a reaction product thereof emits said detectable signal.

2. A device as defined in claim 1 wherein said label comprises an enzyme.

3. A device as defined in claim 2 wherein said enzyme label is reactable to produce a color and said second zone contains material capable of reacting with said enzyme label to produce a color as said detectable signal.

4. A device as defined in claim 3 wherein said substance comprises a fluorophor or a chemiluminescent material and said second zone contains a reagent reactable therewith to emit visible light as said detectable signal.

5. A device as defined in claim 1 wherein said analyte comprises at least one antigen.

6. An assay product for determining the presence of analyte in a sample, said device comprising, in order, the following essential layers:

a first layer containing mobile and diffusible Fab fragments having linked thereto a label for providing a detectable signal or which is the precursor for forming a substance providing a detectable signal;

a second layer containing immobilized reference antigens, said Fab fragments being immuno- logically reactable with said analyt and with said reference antigens; and

a third layer to which the reaction product of said analyte and said labeled Fab fragments can diffuse and in which said label or a reaction product thereof emits said detectable signal, said essential layers being in fluid contact with one another wherein liquid sample applied to the surface of said first layer can permeate therethrough to said third layer.

7. A product as defined in claim 6 including means for applying said liquid sample to the surface of said first layer.

8. A product as defined in claim 6 wherein said essential layers are carried on a transparent support disposed closest to said third layer and through which said detectable signal can be discerned.

9. A product as defined in claim 6 wherein said analyte comprises at least one antigen.

10. A product as defined in claim 6 wherein said label comprises an enzyme.

11. A produce as defined in claim 10 wherein said enzyme label is reactable to produce a color and said third layer contains a chromogenic material reactable with said enzyme label to produce said color as said detectable signal.

12. A product as defined in claim 6 wherein said label is a substance which is reactable with reagent in said third layer to produce electromagnetic radiation as said detectable signal.

0173375

13.   A product as defined in claim 12 wherein said substance comprises a fluorophor.

14.   A product as defined in claim 12 wherein said substance is a chemiluminescent material.

15.   A method for determining the presence of analyte in a liquid sample which comprises:

(a) bringing said liquid sample into contact with said first zone of a device as defined in any of claims 1 to 5;

(b) allowing said liquid to permeate said device; and

(c) determining the presence or absence of any detectable signal being emitted in said second zone, thereby determining the presence or absence of analyte in said liquid sample.

16.   A method for determining the presence of analyte in a liquid sample comprising the steps of:

(a) bringing said liquid sample into contact with the surface of the first layer of a product according to any of claims 6 to 14;

(b) allowing said liquid to permeate said layers of said product; and

(c) determining the presence or absence of any detectable signal being emitted in said third layer, thereby determining the presence or absence of analyte in said liquid sample.

FIG. 1

FIG. 2

FIG. 3

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| D,Y | US-A-4 446 232 (L.A. LIOTTA) <br> * figures 1-6; page 2, paragraph 2 * | 1-7,16 | G 01 N 33/52 <br> G 01 N 33/563 |
| D,Y | US-A-4 235 869 (M. SCHWARZBERG) <br> * page 2, line 29 - page 3, line 40 * | 1-7,16 | |
| A | US-A-4 459 358 (C.M. BERKE) <br> * columns 3,4, complete * | 1,16 | |
| A | US-A-4 298 593 (C.-M. LING) | | |

TECHNICAL FIELDS SEARCHED (Int. Cl.4)

G 01 N 33/00

The present search report has been drawn up for all claims

| Place of search <br> BERLIN | Date of completion of the search <br> 16-10-1985 | Examiner <br> GREEN C.H. |
|---|---|---|